# EUROPÄISCHE PATENTSCHRIFT

(11) **EP 0 897 417 B1**
(45) Veröffentlichungstag und Bekanntmachung des Hinweises auf die Patenterteilung: **24.10.2001**
(21) Anmeldenummer: 97923844.1
(22) Anmeldetag: 05.05.1997
(51) Int. Cl.: C09K 5/04

(54) **WÄSSRIGE KALIUMFORMIAT/KALIUMCARBONATLÖSUNG**
AQUEOUS POTASSIUM FORMATE/ POTASSIUM CARBONATE SOLUTION
SOLUTION AQUEUSE DE FORMIATE DE POTASSIUM/CARBONATE DE POTASSIUM

(30) Priorität: 07.05.1996 DE 19618267
(43) Veröffentlichungstag der Anmeldung: 24.02.1999
(73) Patentinhaber: Linde Aktiengesellschaft, 65189 Wiesbaden (DE); Tyforop Chemie GmbH, 22305 Hamburg (DE)
(72) Erfinder: MALL, Klaus, D-50389 Wesseling (DE); SIERIG, Gisela, D-39122 Magdeburg (DE); HILLERNS, Frank, D-20257 Hamburg (DE)
(86) Internationale Anmeldenummer: EP9702275
(87) Internationale Veröffentlichungsnummer: WO9742272

(56) Entgegenhaltungen:
- EP-A- 0 677 563
- WO-A-93/09198
- DE-A- 3 205 094
- US-A- 4 008 310
- US-A- 5 065 598

## Beschreibung

Die Erfindung betrifft eine wässrige Kaliumformiatlösung, bei der die Salzkonzentration der wässrigen Kaliumformiatlösung zwischen 20 und 55 %-Massenanteil beträgt, und der Kaliumcarbonat zugesetzt ist. Femer betrifft die Erfindung die Verwendung einer derartigen Kaliumformiatlösung als Kälteträger.

Fluorchlorkohlenwasserstoffe (FCKW) galten aufgrund ihrer vorteilhaften physikalischen und physiologischen Eigenschaften seit mehreren Jahrzehnten als unentbehrliche Stoffe für die verschiedensten technischen Anwendungen, wobei sie in der Kältetechnik bevorzugt als Kältemittel für gewerbliche und industrielle Kälteanlagen eingesetzt wurden. Aufgrund der mit ihnen verbundenen Problematik im Hinblick auf die Ozonschicht sowie den Treibhauseffekt, wird seit einigen Jahren verstärkt nach Ersatzstoffen, die ansonsten die gleichen Vorteile aufweisen, gesucht.

Bei industriellen Kälteanlagen hat sich Ammoniak, ein Stoff der weder ein Ozonschädigendes Potential noch ein Treibhauspotential aufweist, seit langem bewährt. Wegen seiner umweltfreundlichen Eigenschaften wird Ammoniak neuerdings auch in Gewerbekälteanlagen verwendet. Zwischenzeitlich wird auch der Einsatz von Kohlenwasserstoffen, wie Propan, Isobutan bzw. daraus hergestellte Mischungen und Propen, als Kältemittel diskutiert. Aufgrund der Giftigkeit von Ammoniak bzw. Brennbarkeit der Kohlenwasserstoffe ist für die zentrale Kälteversorgung in den Verkaufsräumen von Supermärkten die Zwischenschaltung eines Kälteträgers bzw. Kälteträgerkreislaufs erforderlich.

An die innerhalb der Kälteanlagen eingesetzten Kälteträger werden die unterschiedlichsten Anforderungen gestellt. So muss z. B. ein Kälteträger, der in einem Lebensmittelmarkt zum Einsatz kommt, toxisch und ökologisch unbedenklich sein. Femer muss er einen entsprechend hohen Flammpunkt besitzen, um nicht aufwendige Schutzmaßnahmen für den Brandfall zu erfordern. Darüber hinaus sollte die kinematische Viskosität innerhalb des Temperaturbereiches, in dem der Kälteträger zur Anwendung kommt, möglichst gering sein, um einen Energiemehraufwand beim Umpumpen des Kälteträgers und einen nachteiligen Einfluß beim Wärmeübergang zu vermeiden.

Abhängig von den in den Kühlmöbeln vorgesehenen Temperaturbereichen, erfolgt die Kälteübertragung mittels eines Normalkühl-Kreislaufes z. B. bis -15°C und eines Tiefkühl-Kreislaufes z. B. bis -55°C.

Aus der EP-AP 0 677 563 ist ein Verfahren zum Betreiben einer Kälteanlage bekannt, bei dem als Kälteträger eine wässrige Kaliumformiatlösung verwendet wird. Aufgrund der oftmals großen Rohrleitungsnetze von Supermärkten ist bei der Wahl der Kälteträger deren Viskosität zu beachten. Eine höhere Viskosität führt dazu, dass der Energieaufwand beim Umpumpen des Kälteträgers ansteigt Kaliumformiat besitzt gegenüber allen anderen als Kälteträgem verwendeten Salzen, z.B. NaCl, CaCl₂ oder CaCl₂/MgCl₂, eine wesentlich geringere kinematische Viskosität

Kaliumformiat weist im Vergleich zu anderen Kälteträgem in toxikologischer und ökologischer Hinsicht keine Nachteile auf. Es fällt nicht unter die GefahrstoffVerordnung und ist nicht kennzeichnungspflichtig. Es ist femer nach dem Sicherheitsdatenblatt biologisch gut abbaubar; weiterhin unterliegt es nicht der Verordnung der brennbaren Flüssigkeiten (VbF).

Bei der Verwendung von Kaliumformiat in gewerblichen Kälteanlagen im Normalkühlbereich beträgt die Salzkonzentration bei einer Kälteträgertemperatur von -15 bis -30°C zwischen 30 und 40 %-Massenanteil. Bei einer Verwendung im Tiefkühlbereich, also bei einer Kälteträgertemperatur von -30 bis ca. -55°C, beträgt die Salzkonzentration zwischen 40 und ca. 55 %-Massenanteil.

Als Korrosions-Inhibitoren können der wässrigen Kaliumformiatlösung 1,2,4-Triazol sowie alternativ oder zusätzlich Borax zugesetzt werden. Damit läßt sich bei allen bekannten Werkstoffen eine hervorragende Korrosionsinhibierung erzielen. Bei Gusseisen, wie z .B. GG 25, kann jedoch keine ausreichende Qualität hinsichtlich der Korrosionsinhibierung erzielt werden.

Aus der SU-A-305 952 ist eine wässrige Lösung, bestehend aus Kaliumformiat und Kaliumcarbonat bekannt; diese Lösung wird jeodch nur als Schmiermittel verwendet. Die Anforderung an Schmiermittel und Kälteträger sind jedoch unterschiedlich.

Aufgabe der vorliegenden Erfindung ist es, einen Kälteträger auf der Basis einer wässrigen Kaliumformiatlösung anzugeben, der auch in Anwesenheit des Werkstoffes Gusseisen eine gute Korrosionsinhibierung gewährleistet.

Diese Aufgabe wird erfindungsgemäß dadurch gelöst, dass als Kälteträger eine wässrige Kaliumformiatlösung, bei der die Salzkonzentration der wässrigen Kaliumformiatlösung zwischen 20 und 55 %-Massenanteil beträgt, und der Kaliumcarbonat zugesetzt ist, verwendet wird.

Die wässrige Kaliumformiatlösung, bei der die Salzkonzentration der wässrigen Kaliumformiatlösung zwischen 20 und 55 %-Massenanteil beträgt, und der Kaliumcarbonat zugesetzt ist, zeichnet sich dadurch aus, dass bei einer Kälteträgertemperatur von -15 bis -30°C die Salzkonzentration des Kaliumformiats zwischen 25 und 30 %-Massenanteil und die Salzkonzentration des Kaliumcarbonats zwischen 7 und 10 %-Massenanteil beträgt.

Durch die Zugabe von Kaliumcarbonat zu der wässrigen Kaliumformiatlösung ist auch bei der Verwendung von Gußeisen eine gute Korrosionsinhibierung gewährleistet. Eine wesentliche Verschlechterung der positiven Eigenschaften der reinen wässrigen Kaliumformiatlösung tritt durch die Zugabe von Kaliumcarbonat bei den dafür verwendeten Mischungsanteilen der Salze nicht ein.

Eine Ausgestaltung der Erfindung ist dadurch gekennzeichnet, daß bei einer Kälteträgertemperatur von -15 bis -30°C die Salzkonzentration des Kaliumformiats zwischen 20 und 35 %-Massenanteil, vorzugsweise zwischen 25 und 30 %-Massenanteil, und die Salzkonzentration des Kaliumcarbonats zwischen 5 und 15 %-Massenanteil, vorzugsweise zwischen 7 und 10 %-Massenanteil, beträgt, während bei einer Kälteträgertemperatur von -30 bis -55°C die Salzkonzentration des Kaliumformiats zwischen 25 und 55 %-Massenanteif, vorzugsweise zwischen 30 und 40 %-Massenanteil, und die Salzkonzentration des Kaliumcarbonats zwischen 5 und 15 %-Massenanteil, vorzugsweise zwischen 6 und 11 %-Massenanteil, beträgt.

Es kann notwendig sein, daß der Kälteträger z.B. dann, wenn er auch zum Abtauen von Kälteanlagen verwendet wird, zumindest kurzfristig Temperaturen bis ca. 80°C standhalten kann.

Die Erfindung weiterbildend wird daher vorgeschlagen, daß dem erfindungsgemäßen Kälteträger als Inhibitor Mono- und/oder Polysaccharide und/oder deren Alkalisalze in einer Konzentration zwischen 500 und 5000 ppm zugesetzt werden. Dadurch wird erreicht, daß auch in diesen Temperaturbereichen eine optimale Korrosionsinhibierung gewährleistet werden kann.

Wird der erfindungsgemäße Kälteträger in einer Anlage, in der neben Gussmaterialien z.B. Rohrleitungen und/oder Wärmetauscher aus bzw. mit Kupfer vorgesehen sind, verwendet, so empfiehlt es sich, gemäß einer vorteilhaften Ausgestaltung der Erfindung, als Inhibitor 1,2,4-Triazol in einer Konzentration zwischen 10 und 2000 ppm, vorzugsweise zwischen 200 und 1500 ppm, zuzusetzen.

Alternativ oder zusätzlich dazu können als Kupfer-Inhibitoren Benzotriazol oder Tolyltriazol in einer Konzentration zwischen 10 und 2000 ppm, vorzugsweise zwischen 200 und 1500 ppm, zugesetzt werden.

Desweiteren kann, gemäß einer vorteilhaften Ausgestaltung der Erfindung, als Inhibitor Borax in einer Konzentration zwischen 500 und 2500 ppm zugesetzt werden. Die Zusetzung von Borax dient der Inhibierung bei der Verwendung von Stahl- und/oder Graugußmaterialien sowie der pH-Pufferung.

Um kleinste Undichtigkeiten in Rohrleitungen, Armaturen oder Apparaten erkennen zu können, wird ferner vorgeschlagen, daß dem erfindungsgemäßen Kälteträger ein Farbstoff, wie z.B. Xylenolorange in einer Konzentration von 10 bis 50 ppm, vorzugsweise von 20 bis 40 ppm, zugesetzt wird.

Mit dem erfindungsgemäßen Kälteträger steht nunmehr ein Kälteträger zur Verfügung, der auch bei der Verwendung von Guß-Materialien in Kälteträgerkreis-läufen eine optimale Inhibierung gewährleistet. Durch die Zugabe geeigneter Inhibitoren kann diese optimale Inhibierung bei der Verwendung von Guß-Materialien in Kälteträgerkreisläufen in allen für die Praxis relevanten Temperaturbereichen realisiert werden.

## Patentansprüche

1. Verwendung einer wässrigen Kaliumformiatlösung, bei der die Salzkonzentration der wässrigen Kaliumformiatlösung zwischen 20 und 55 %-Massenanteil beträgt, und der Kaliumcarbonat zugesetzt ist, als Kälteträger.

2. Verwendung einer wässrigen Kaliumformiatlösung als Kälteträger nach Anspruch 1, **dadurch gekennzeichnet, daß** bei einer Kälteträgertemperatur von -15 bis -30 °C die Salzkonzentration des Kaliumformiats zwischen 20 und 35 %-Massenanteil und die Salzkonzentration des Kaliumcarbonats zwischen 5 und 15 %-Massenanteil beträgt.

3. Verwendung einer wässrigen Kaliumformiatlösung als Kälteträger nach Anspruch 2, **dadurch gekennzeichnet, daß** bei einer Kälteträgertemperatur von -15 bis -30°C die Salzkonzentration des Kaliumformiats zwischen 25 und 30 %-Massenanteil und die Salzkonzentration des Kaliumcarbonats zwischen 7 und 10 %-Massenanteil beträgt.

4. Verwendung einer wässrigen Kaliumformiatlösung als Kälteträger nach Anspruch 1, **dadurch gekennzeichnet, daß** bei einer Kälteträgertemperatur von -30 bis -55°C die Salzkonzentration des Kaliumformiats zwischen 25 und 55 %-Massenanteil und die Salzkonzentration des Kaliumcarbonats zwischen 5 und 15 %-Massenanteil beträgt.

5. Verwendung einer wässrigen Kaliumformiatlösung als Kälteträger nach Anspruch 4, **dadurch gekennzeichnet, daß** bei einer Kälteträgertemperatur von -30 bis -55°C die Salzkonzentration des Kaliumformiats zwischen 30 und 40 %-Massenanteil und die Salzkonzentration des Kaliumcarbonats zwischen 6 und 11 %-Massenanteil beträgt.

6. Verwendung einer wässrigen Kaliumformiatlösung als Kälteträger nach einem der vorhergehenden Ansprüche, **dadurch gekennzeichnet, daß** die wässrige Kaliumformiatlösung als Korrosionsinhibitor Mono- und/oder Polysaccharide und/oder deren Alkalisalze in einer Konzentration zwischen 500 und 5000 ppm enthält.

7. Verwendung einer wässrigen Kaliumformiatlösung als Kälteträger nach einem der vorhergehenden Ansprüche, **dadurch gekennzeichnet, daß** die wässrige Kaliumformiatlösung als Inhibitor 1,2,4-Triazol in einer Konzentration zwischen 10 und 2000 ppm enthält.

8. Verwendung einer wässrigen Kaliumformiatlösung als Kälteträger nach einem der vorhergehenden Ansprüche, **dadurch gekennzeichnet, daß** die wässrige Kaliumformiatlösung als Inhibitor Benzotriazol oder Tolyltriazol in einer Konzentration zwischen 10 und 2000 ppm enthält

9. Verwendung einer wässrigen Kaliumformiatlösung als Kälteträger nach einem der vorhergehenden Ansprüche, **dadurch gekennzeichnet, daß** die wässrige Kaliumformiatlösung als Inhibitor Borax in einer Konzentration zwischen 500 und 2500 ppm enthält

10. Verwendung einer wässrigen Kaliumformiatlösung als Kälteträger nach einem der vorhergehenden Ansprüche, **dadurch gekennzeichnet, daß** die wässrige Kaliumformiatlösung einen Farbstoff in einer Konzentration von 10 bis 50 ppm enthält.

11. Wässrige Kaliumformiatlösung, bei der die Salzkonzentration der wässrigen Kaliumformiatlösung zwischen 20 und 55 %-Massenanteil beträgt, und der Kaliumcarbonat zugesetzt ist, **dadurch gekennzeichnet, daß** bei einer Kälteträgertemperatur von -15 bis -30°C die Salzkonzentration des Kaliumformiats zwischen 25 und 30 %-Massenanteil und die Salzkonzentration des Kaliumcarbonats zwischen 7 und 10 %-Massenanteil beträgt

12. Wässrige Kaliumformiatlösung nach Anspruch 11, **dadurch gekennzeichnet, daß** bei einer Kälteträgertemperatur von -30 bis -55°C die Salzkonzentration des Kaliumformiats zwischen 25 und 55 %-Massenanteil und die Salzkonzentration des Kaliumcarbonats zwischen 5 und 15 %-Massenanteil beträgt

13. Wässrige Kaliumformiatlösung nach Anspruch 12, **dadurch gekennzeichnet, daß** bei einer Kälteträgertemperatur von -30 bis -55°C die Salzkonzentration des Kaliumformiats zwischen 30 und 40 %-Massenanteil und die Salzkonzentration des Kaliumcarbonats zwischen 6 und 11 %-Massenanteil beträgt.

14. Wässrige Kaliumformiatiösung nach einem der Ansprüche 11 bis 13, **dadurch gekennzeichnet, daß** die wässrige Kaliumformiatiösung als Korrosionsinhibitor Mono- und/oder Polysaccharide und/oder deren Alkalisalze in einer Konzentration zwischen 500 und 5000 ppm enthält.

15. Wässrige Kaliumformiatlösung nach einem der Ansprüche 11 bis 14, **dadurch gekennzeichnet, daß** die wässrige Kaliumformiatlösung als Inhibitor 1,2,4-Triazol in einer Konzentration zwischen 10 und 2000 ppm enthält.

16. Wässrige Kaliumformiatlösung nach einem der Ansprüche 11 bis 15, **dadurch gekennzeichnet, daß** die wässrige Kaliumformiatlösung als Inhibitor Benzotriazol oder Tolyltriazol in einer Konzentration zwischen 10 und 2000 ppm enthält.

17. Wässrige Kaliumformiatiösung nach einem der Ansprüche 11 bis 16, **dadurch gekennzeichnet, daß** die wässrige Kaliumformiatlösung als Inhibitor Borax in einer Konzentration zwischen 500 und 2500 ppm enthält.

18. Wässrige Kaliumformiatiösung nach einem der Ansprüche 11 bis 17, **dadurch gekennzeichnet, daß** die wässrige Kaliumformiatlösung einen Farbstoff in einer Konzentration von 10 bis 50 ppm enthält.

## Claims

1. Use of an aqueous potassium formate solution as refrigerant, in which the salt concentration of the aqueous potassium formate solution is between 20 and 55% by mass and to which potassium carbonate is added.

2. Use of an aqueous potassium formate solution as refrigerant according to Claim 1, **characterized in that**, at a refrigerant temperature of -15 to -30°C, the salt concentration of the potassium formate is between 20 and 35% by mass and the salt concentration of the potassium carbonate is between 5 and 15% by mass.

3. Use of an aqueous potassium formate solution as refrigerant according to Claim 2, **characterized in that**, at a refrigerant temperature of -15 to -30°C, the salt concentration of the potassium formate is between 25 and 30% by mass and the salt concentration of the potassium carbonate is between 7 and 10% by mass.

4. Use of an aqueous potassium formate solution as refrigerant according to Claim 1, **characterized in that**, at a refrigerant temperature of -30 to -55°C, the salt concentration of the potassium formate is between 25 and 55% by mass and the salt concentration of the potassium carbonate is between 5 and 15% by mass.

5. Use of an aqueous potassium formate solution as refrigerant according to Claim 4, **characterized in that**, at a refrigerant temperature of -30 to -55°C, the salt concentration of the potassium formate is between 30 and 40% by mass and the salt concentration of the potassium carbonate is between 6 and 11% by mass.

6. Use of an aqueous potassium formate solution as refrigerant according to one of the preceding claims, **characterized in that** the aqueous potassium formate solution comprises, as corrosion inhibitor, mono- and/or polysaccharides and/or their alkaline metal salts at a concentration between 500 and 5000 ppm.

7. Use of an aqueous potassium formate solution as refrigerant according to one of the preceding claims, **characterized in that**, the aqueous potassium formate solution comprises as inhibitor 1,2,4-triazole at a concentration between 10 and 2000 ppm.

8. Use of an aqueous potassium formate solution as refrigerant according to one of the preceding claims, **characterized in that** the aqueous potassium formate solution comprises as inhibitor benzotriazole or tolyltriazole at a concentration between 10 and 2000 ppm.

9. Use of an aqueous potassium formate solution as refrigerant according to one of the preceding claims, **characterized in that** the aqueous potassium formate solution comprises as inhibitor borax at a concentration between 500 and 2500 ppm.

10. Use of an aqueous potassium formate solution as refrigerant according to one of the preceding claims, **characterized in that** the aqueous potassium formate solution comprises a dye at a concentration of 10 to 50 ppm.

11. Aqueous potassium formate solution in which the salt concentration of the aqueous potassium formate solution is between 20 and 55% by mass and to which potassium carbonate is added, **characterized in that**, at a refrigerant temperature of -15 to -30°C, the salt concentration of the potassium formate is between 25 and 30% by mass and the salt concentration of the potassium carbonate is between 7 and 10% by mass.

12. Aqueous potassium formate solution according to Claim 11, **characterized in that**, at a refrigerant temperature of -30 to -55°C, the salt concentration of the potassium formate is between 25 and 55% by mass and the salt concentration of the potassium carbonate is between 5 and 15% by mass.

13. Aqueous potassium formate solution according to Claim 12, **characterized in that**, at a refrigerant temperature of -30 to -55°C, the salt concentration of the potassium formate is between 30 and 40% by mass and the salt concentration of the potassium carbonate is between 6 and 11% by mass.

14. Aqueous potassium formate solution according to one of Claims 11 to 13, **characterized in that** the aqueous potassium formate solution comprises, as corrosion inhibitor, mono- and/or polysaccharides and/or their alkaline metal salts at a concentration between 500 and 5000 ppm.

15. Aqueous potassium formate solution according to one of Claims 11 to 14, **characterized in that** the aqueous potassium formate solution comprises as inhibitor 1,2,4-triazole at a concentration between 10 and 2000 ppm.

16. Aqueous potassium formate solution as claimed in one of Claims 11 to 15, **characterized in that** the aqueous potassium formate solution comprises as inhibitor benzotriazole or tolyltriazole at a concentration between 10 and 2000 ppm.

17. Aqueous potassium formate solution according to one of Claims 11 to 16, **characterized in that** the aqueous potassium formate solution comprises as inhibitor borax at a concentration between 500 and 2500 ppm.

18. Aqueous potassium formate solution as claimed in one of Claims 11 to 17 **characterized in that**, the aqueous potassium formate solution comprises a dye at a concentration of 10 to 50 ppm.

## Revendications

1. Utilisation d'une solution aqueuse de formiate de potassium, dans laquelle la concentration en sel de la solution aqueuse de formiate de potassium est comprise entre 20 et 55% en proportion massique, et le carbonate de potassium est ajouté en tant que caloporteur.

2. Utilisation d'une solution aqueuse de formiate de potassium en tant que caloporteur selon la revendication 1, **caractérisée en ce que**, à une température du caloporteur de -15 à -30°C, la concentration en sel du formiate de potassium est comprise entre 20 et 35% en poids en proportion massique et la concentration en sel du carbonate de potassium est comprise entre 5 et 15% en proportion massique.

3. Utilisation d'une solution aqueuse de formiate de potassium en tant que caloporteur selon la revendication 2, **caractérisée en ce que**, à une température du caloporteur de -15 à -30°C, la concentration en sel du formiate de potassium est comprise entre 25 et 30% en poids en proportion massique et la concentration en sel du carbonate de potassium est comprise entre 7 et 10% en proportion massique.

4. Utilisation d'une solution aqueuse de formiate de potassium en tant que caloporteur selon la revendication 1, **caractérisée en ce que**, à une température du caloporteur de -30 à -55°C, la concentration en sel du formiate de potassium est comprise entre 25 et 55% en poids en proportion massique et la concentration en sel du carbonate de potassium est comprise entre 5 et 15% en proportion massique.

5. Utilisation d'une solution aqueuse de formiate de potassium en tant que caloporteur selon la revendication 4, **caractérisée en ce que**, à une température du caloporteur de -30 à -55°C, la concentration en sel du formiate de potassium est comprise entre 30 et 40% en poids en proportion massique et la concentration en sel du carbonate de potassium est comprise entre 6 et 11% en proportion massique.

6. Utilisation d'une solution aqueuse de formiate de potassium en tant que caloporteur selon l'une quelconque des revendications précédentes, **caractérisée en ce que** la solution aqueuse de formiate de potassium contient, en tant qu'inhibiteur de corrosion, des mono- et/ou polysaccharides et/ou leurs sels de métaux alcalins en une concentration comprise entre 500 et 5 000 ppm.

7. Utilisation d'une solution aqueuse de formiate de potassium en tant que caloporteur selon l'une quelconque des revendications précédentes, **caractérisée en ce que** la solution aqueuse de formiate de potassium contient, en tant qu'inhibiteur, du 1,2,4-triazole en une concentration comprise entre 10 et 2 000 ppm.

8. Utilisation d'une solution aqueuse de formiate de potassium en tant que caloporteur selon l'une quelconque des revendications précédentes, **caractérisée en ce que** la solution aqueuse de formiate de potassium contient, en tant qu'inhibiteur, du benzotriazole ou du tolyltriazole en une concentration comprise entre 10 et 2 000 ppm.

9. Utilisation d'une solution aqueuse de formiate de potassium en tant que caloporteur selon l'une quelconque des revendications précédentes, **caractérisée en ce que** la solution aqueuse de formiate de potassium contient, en tant qu'inhibiteur, du borax en une concentration comprise entre 500 et 2 500 ppm.

10. Utilisation d'une solution aqueuse de formiate de potassium en tant que caloporteur selon l'une quelconque des revendications précédentes, **caractérisée en ce que** la solution aqueuse de formiate de potassium contient un colorant en une concentration de 10 à 50 ppm.

11. Solution aqueuse de formiate de potassium, dans laquelle la concentration en sel de la solution aqueuse de formiate de potassium est comprise entre 20 et 55% en proportion massique et le carbonate de potassium est ajouté, **caractérisée en ce que**, à une température du caloporteur de -15 à -30°C, la concentration en sel du formiate de potassium est comprise entre 25 et 30% en proportion massique et la concentration en sel du carbonate de potassium est comprise entre 7 et 10% en proportion massique.

12. Solution aqueuse de formiate de potassium selon la revendication 11, **caractérisée en ce que**, à une température du caloporteur de -30 à -55°C, la concentration en sel du formiate de potassium est comprise entre 25 et 55% en poids en proportion massique et la concentration en sel du carbonate de potassium est comprise entre 5 et 15% en proportion massique.

13. Solution aqueuse de formiate de potassium selon la revendication 12, **caractérisée en ce que**, à une température du caloporteur de -30 à -55°C, la concentration en sel du formiate de potassium est comprise entre 30 et 40% en poids en proportion massique et la concentration en sel du carbonate de potassium est comprise entre 6 et 11% en proportion massique.

14. Solution aqueuse de formiate de potassium selon l'une quelconque des revendications 11 à 13, **caractérisée en ce que** la solution aqueuse de formiate de potassium contient, en tant qu'inhibiteur de corrosion, des mono- et/ou polysaccharides et/ou leurs sels de métaux alcalins en une concentration comprise entre 500 et 5 000 ppm.

15. Solution aqueuse de formiate de potassium selon l'une quelconque des revendications 11 à 14, **caractérisée en ce que** la solution aqueuse de formiate de potassium contient, en tant qu'inhibiteur, du 1,2,4-triazole en une concentration comprise entre 10 et 2 000 ppm.

16. Solution aqueuse de formiate de potassium selon l'une quelconque des revendications 11 à 15, **caractérisée en ce que** la solution aqueuse de formiate de potassium contient, en tant qu'inhibiteur, du benzotriazole ou du tolyltriazole en une concentration comprise entre 10 et 2 000 ppm.

17. Solution aqueuse de formiate de potassium selon l'une quelconque des revendications 11 à 16, **caractérisée en ce que** la solution aqueuse de formiate de potassium contient, en tant qu'inhibiteur, du borax en une concentration comprise entre 500 et 2 500 ppm.

18. Solution aqueuse de formiate de potassium selon l'une quelconque des revendications 11 à 17, **caractérisée en ce que** la solution aqueuse de formiate de potassium contient un colorant en une concentration de 10 à 50 ppm.
